# EUROPEAN PATENT SPECIFICATION

(11) **EP 0 437 290 B1**
(45) Date of publication and mention of the grant of the patent: **06.05.1998**
(21) Application number: 91200009.8
(22) Date of filing: 04.01.1991
(51) Int. Cl.: C07C 255/17, C07C 253/30, C07D 213/09

(54) **Process for the preparation of 5-oxohexane nitriles and the compound 2,4-dimethyl-5-oxohexane nitrile**
Verfahren zur Herstellung von 5-oxohexannitrilen und die Verbindung 2,4-dimethyl-5-oxo-hexannitrile
Procédé pour la préparation de 5-oxohexanenitriles et composé 2,4-diméthyl-5-oxohexanenitrile

(30) Priority: 06.01.1990 NL 9000034
(43) Date of publication of application: 17.07.1991
(73) Proprietor: DSM N.V., 6411 TE Heerlen (NL)
(72) Inventor: Castelijns, Anna Maria Cornelia Francisca, NL-6171 JG Stein (L.) (NL); Arts, Henricus Johannes, NL-6135 CW Sittard (NL); Green, Richard, NL-6163 KD Geleen (NL)

(56) References cited:
- FR-A- 2 089 392
- US-A- 2 381 371
- US-A- 2 579 580
- US-A- 2 850 519
- US-A- 3 816 503
- US-A- 4 117 000
- US-A- 4 245 098
- US-A- 4 267 362
- US-A- 4 405 552
- US-A- 4 478 354
- US-A- 4 996 323
- JOURNAL OF THE AMERICAN CHEMICAL SOCIETY, vol. 75, no. 12, 24th june 1953, pages 3015-3016; H.E. BAUMGARTEN et al.: "Monocyanoethylation of certain ketones"

## Description

The invention relates to a process for the preparation of 5-oxohexane nitriles according to Formula 1, 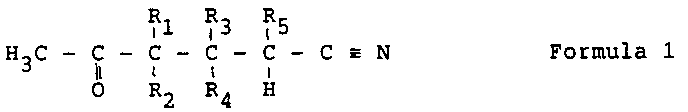 where at least R₁ or R₂ is not hydrogen, by catalytic conversion of a methyl ketone according to Formula 2 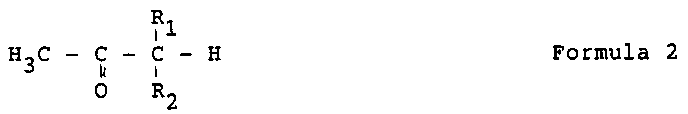 where R₁ and R₂ denote the same as in Formula 1, and an α-β unsaturated nitrile according to Formula 3

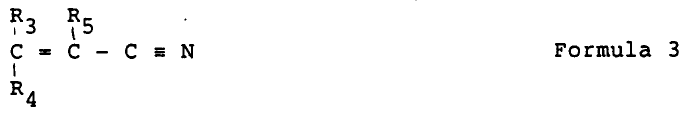

Such a process is know from US patent specification US-A-2850519. In the said publication the reaction of acrylic acid nitrile and ketones in the presence of primary amines as catalyst is described. It has appeared, however, that when in the said process asymmetrical methyl ketones are used as starting material in combination with other nitriles than acrylic acid nitrile, e.g. methacrylic acid nitrile, isomer mixtures are formed that contain a substantial amount of undesired isomer, namely the product of addition of the α-β unsaturated nitrile to the methyl group of the ketone, whereas this is not the case when acrylic acid nitrile is used as starting material in such a reaction. Isolation of the isomers generally is very difficult.

As a consequence, the yield in which the 5-oxo-hexane nitriles according to Formula 1 can be obtained in purified form, that is, with less than 0.2 wt. % of undesired isomer, generally is very low, a major part of the nitrile being lost in the separation process.

The process according to the invention aims to sub-stantially avoid the formation of undesired isomers.

According to the invention this is achieved when R₁ and R₂ each independently are H, alkyl or alkoxy with 1-20 carbon atoms provided that not both R₁ and R₂ are H and when R₃, R₄ and R₅, each independently are H, alkyl cycloalkyl or alkoxy with 1-20 C-atoms, provided that R₃, R₄ and R₅ are not all hydrogen, by using a strong base as a catalyst.

The use of a strong base in general as a catalyst for this type of reactions in particular in cyanoethylation reactions with acrylic acid nitrile, is known per se. However, it was generally assumed, as e.g. is illustrated in US-A-2850519 that the use of a strong base is disadvantageous since the yield of the desired product would be low due to side reactions such as, for instance, aldol condensation of ketones, and the formation of polycyanoethylated products when the ketones have two or more active hydrogen atoms.

The invention furthermore relates to the new compound 2,4-dimethyl-5-oxohexane nitrile.

The US patent specification US-A-4647686 discloses a general method for the preparation of 5-oxohexane nitriles from corresponding cycloalkanones. However, no mention is made of the cyclopentanone used as starting material for 2,4-dimethyl-5-oxohexane nitrile in this method, viz. 2,3,5-trimethyl cyclopentanone.

The US patent specification US-A-2579580 discloses a process wherein a crosslinked polyquaternary ammonium hydroxide resin is used for cyanoethylation reactions in order to prevent to a larger extend the formation of polycyano ethylation products.

The need for a method for the preparation of 5-oxohexane nitriles according to Formula 1, in which at least R₁ or R₂ and at least one of the groups R₃, R₄, or R₅ is not hydrogen, results from the demand for pure 5-oxohexane nitriles among the users of these compounds or of compounds that can be prepared from them. 5-Oxohexane nitriles are used as starting materials for pharmaceutical and agrochemical intermediates and final products.

A specific application of the 5-oxohexane nitriles according to Formula 1, where R₂ and R₄ are hydrogen and at least R₁ and at least R₃ or R₅ is not hydrogen, is the preparation of 2-methyl pyridines according to Formula 4:

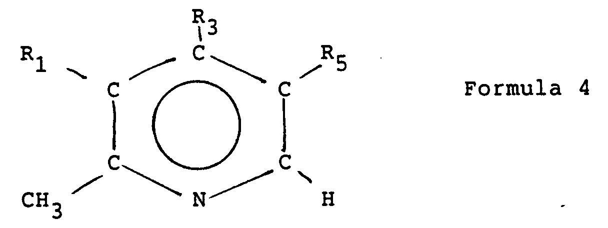

A number of these pyridine derivatives are important starting materials for the preparation of pharmaceutical products. In the preparation of such products often strict demands as regards the purity of the starting materials are to be met. A requirement of less than 0.2 wt. % of undesirable pyridine derivatives is not uncommon.

In many cases it is impossible to effect adequate separation of a mixture of isomeric pyridine derivatives at acceptable costs, certainly when large amounts of undesirable pyridine isomers need to be isolated.

A method for the preparation of the above-mentioned 2-methyl pyridines having a high purity, so that the separation problem is obviated, is the catalytic cyclization and dehydrogenation of 5-oxohexane nitriles according to Formula 1, where R₂ and R₄ are hydrogen and at least R₁ and at least R₃ or R₅ is not hydrogen, according to a process known per se, for instance in the gas phase. A condition to be satisfied for high purity of the final product is that the nitrile must also have a very low content of undesirable isomer.

The process according to the invention can be used to prepare nitriles having a low content of undesirable isomers.

One of the substances that can be prepared using the process according to the invention is the new substance 2,4-dimethyl-5-oxohexane nitrile.
From this substance 2,3,5-trimethyl pyridine can be prepared by means of, for instance, catalytic gas-phase cyclization and dehydrogenation as described in UK patent specification GB-A-1304155.

2,3,5-Trimethyl pyridine among other things is a starting material for pharmaceutical preparations regulating the secretion of gastric acid. For this application, the above-mentioned considerations relating to the purity of the products certainly hold.

Most of the preparation methods for 2,3,5-trimethyl pyridine currently known start from other substituted pyridine derivatives. According to, for instance, US patent specification US-A-4658032 2,3,5-trimethyl pyridine can be prepared from 3,5-lutidine by reacting this substance with an alcohol in the presence of an hydrogenation catalyst at a temperature of 200 °C or higher. A drawback of this method is the very high catalyst consumption. Another drawback of this preparation method, and in general of preparation methods starting from pyridines, substituted or not, is that the pyridines used as starting material themselves often are hard to obtain.

These drawbacks do not present themselves in the preparation of 2,3,5-trimethyl pyridine starting from 2,4-dimethyl-5-oxohexane nitrile.

The major problem in this preparation method was how to obtain the starting material, 2-4-dimethyl-5-oxo-hexane nitrile, in sufficiently pure form. The thusfar known preparation methods which offer the highest yields in cyano-ethylation reactions of ketons with acrylic acid nitrile, such as those described in the above-mentioned US patent specification US-A-2850519, have as byproduct substantial amounts (desirable isomer : undesirable isomer = about 2:1) 2-methyl-5-oxoheptane nitrile:

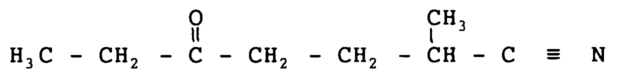

Separation of 2-methyl-5-oxoheptane nitrile and 2,4-dimethyl-5-oxohexane nitrile is very difficult, but it is necessary, because in gas phase cyclization 2-methyl-5-oxoheptane nitrile yields 2-ethyl-5-methyl pyridine as product, which is undesirable and which in addition is difficult to isolate from 2,3,5-trimethyl pyridine.

With the process according to the invention the formation of 2-methyl-5-oxoheptane nitrile during the preparation of 2,4-dimethyl-5-oxohexane nitrile can to a large extent be prevented. Without problems an isomer ratio of about 10:1 can be achieved, as opposed to about 2:1 with the known processes.

The ketones to be used in the process according to the invention are ketones according to Formula 2 wherein R₁ or R₂ are:
alkyl, substituted or not,
cycloalkyl, substituted or not, with 1-20 carbon atoms.
Suitable substituents are substituents that are stable under reaction conditions, such as, for instance, alkoxy, aryl and heteroaryl with 1-10 carbon atoms. Examples of suitable ketones are: butanone, pentane-2-one, hexane-2-one, methoxy-acetone, heptane-2-one, 3-methyl-pentane-2-one, 4-methyl-pentane-2-one, benzyloxyacetone.

The nitriles that can be used in the process are nitriles according to Formula 3 where at least one of the groups R₃, R₄ or R₅ is not hydrogen. Suitable choices for the groups R₃, R₄ and R₅ are:
alkyl, substituted or not,
cycloalkyl, substituted or not,
alkoxy, substituted or not with 1-20 carbon atoms.
Suitable substituents are substituents that are stable under reaction conditions, such as, for instance, alkoxy, aryl and heteroaryl.
These groups will mostly contain fewer than 7 carbon atoms. Examples of nitriles are methacrylic acid nitrile, 2-cyano-1-butene, 2-cyano-1-pentene, 2-cyano-1-hexene, 2-cyano-2-butene, crotonic nitrile, 3-cyclohexyl-2-cyano-1-butene, 2-cyclohexyl propene nitrile.

A suitable way of carrying out the process is as follows:

The ketone, the nitrile, the strong base and optionally an inert solvent are combined. The sequence in which the components are added as well as the feed rates are not critical. The components may be combined before the reaction, but it is also possible to add one or more components entirely or partly during the reaction. The reaction mixture is heated to the reaction temperature and kept at this temperature for some time. Upon completion of the reaction the reaction mixture is, if necessary, cooled to room temperature. The reaction mixture is subsequently neutralized and the organic phase is washed. Non-converted reactants and any solvent present are removed, for instance by evaporation, optionally at reduced pressure, and the product is recovered by distillation, also optionally at reduced pressure.

The molar ratio of ketone/nitrile in general is not critical and can be varied within broad limits. The highest yields are generally obtained when an excess of ketone is used. The ratio of ketone to nitrile preferably is between 2 and 7, more preferably between 3 and 5.

As catalyst in principle any strong base can be used. Examples are alkali alkanolates, alkali hydroxides, tetra-alkyl ammonium hydroxides (e.g. Triton B), alkali hydrides, alkali amides. Very good results are achieved when use is made of tetra-alkyl ammonium hydroxides.

The base can be applied in many ways. Depending on its nature, the base may for instance be applied as a solution in water, a solution in methanol, or in solid form.

The concentration of the base in the organic phase can be varied within broad limits. By varying the concentration the reaction velocity can be influenced; a higher concentration yields a higher velocity.

By preference use is made of sodium hydroxide or potassium hydroxide. If the alkali hydroxide is added as an aqueous solution, this is preferably done in a concentration of more than 10 wt. %, and more particularly in a concentration of more than 40 wt. %.

The use of a base as a solid, for instance sodium hydroxide in powder form, generally results in high reaction velocities. A very suitable form of base application is as a solution in methanol, preferably a saturated solution in methanol. The use of alkali hydroxide as a base then yields the best results.

The temperature during the reaction is not critical. A suitable temperature is the reflux temperature of the reaction mixture. This may vary within broad limits, depending on the mixture. However, in a number of cases it is quite possible to carry out the reaction at room temperature. It also is possible to use higher temperatures when working under elevated pressure in an autoclave. Temperatures below the reflux temperature generally decrease the reaction velocity.

Several methods are suitable for upgrading of the mixture.
One of these is the neutralization of the crude reaction mixture. After this, the reaction mixture can be washed by treating it several times with a salt solution. After these washing treatments the non-converted starting materials and any solvent present can be removed by evaporation, optionally under reduced pressure. The product can then be recovered by distillation.

The process according to the invention will now be elucidated with reference to the following examples, without being restricted thereto.

In the description of the examples and in the tables, the following symbols have the meanings denoted below:
- MACN:: methacrylic acid nitrile
- ACN:: acrylic acid nitrile
- BzOH:: benzoic acid
- A:: 2,4-dimethyl-5-oxohexane nitrile
- B:: 2-methyl-5-oxoheptane nitrile
- C:: 4-methyl-5-oxohexane nitrile
- D:: 5-oxoheptane nitrile
- conv.:: conversion
- sel.:: selectivity
- t:: reaction time
- T:: temperature
- m.r.:: molar ratio

### Example I

### Ia. Preparation of A.

288.0 g of butanone (4.0 mol) and 67 g of MACN (1.0 mol) were introduced into a 500 ml round bottom flask, equipped with a thermometer, cooler and stirrer. With stirring, 8.75 g of a 15 wt.% NaOH solution in methanol (0.033 mol NaOH) was added. The reaction mixture was heated up to reflux (heating time approx. 14 minutes, reflux temperature 81 °C). After a total reaction time of 90 minutes the mixture was cooled to room temperature. Gas chromatographic analysis showed that about 99.0% of the MACN supplied has been converted, and about 25.7% of the butanone supplied. Calculated relative to converted MACN, the yield in terms of A is 36.4% and in terms of B 3.4%. Calculated relative to converted butanone these yields are approximately 30.5% and 2.9%, respectively.
(Ratio of A : B = 10.6: 1). The result is presented in Table 1.

### Ib. Purification

The reaction mixture was subsequently neutralized using 16.1 g of 10% H₂SO₄ solution in H₂O. Then 50 ml of a 10 wt.% solution of Na₂SO₄ in water was added. After demixing of the two phases, the water phase was separated off and the organic phase was washed with 50 ml of a 10 wt.% Na₂SO₄ solution. Of the organic phase the excess butanone was evaporated using the rotavapor, the residue being subjected to vacuum distillation using a packed column with approx. 40 practical trays. At a pressure of 15 mm Hg the main fraction came off at the top with a temperature of 110-113 °C. This way 40.75 g of distillate was obtained. Gas chromatographic analysis showed this distillate to contain 40.1 g of A and 0.04 g of B (ratio of A : B = 1000: 1).

### Examples II to IX

To a mixture of butanone and MACN an amount of strong base was added, optionally in the form of solution. The reaction mixture was then brought at a certain temperature during a number of hours. The mixture was subsequently analyzed gas chromatographically. The procedure followed was analogous to that of Example Ia. The results are presented in Table 1.

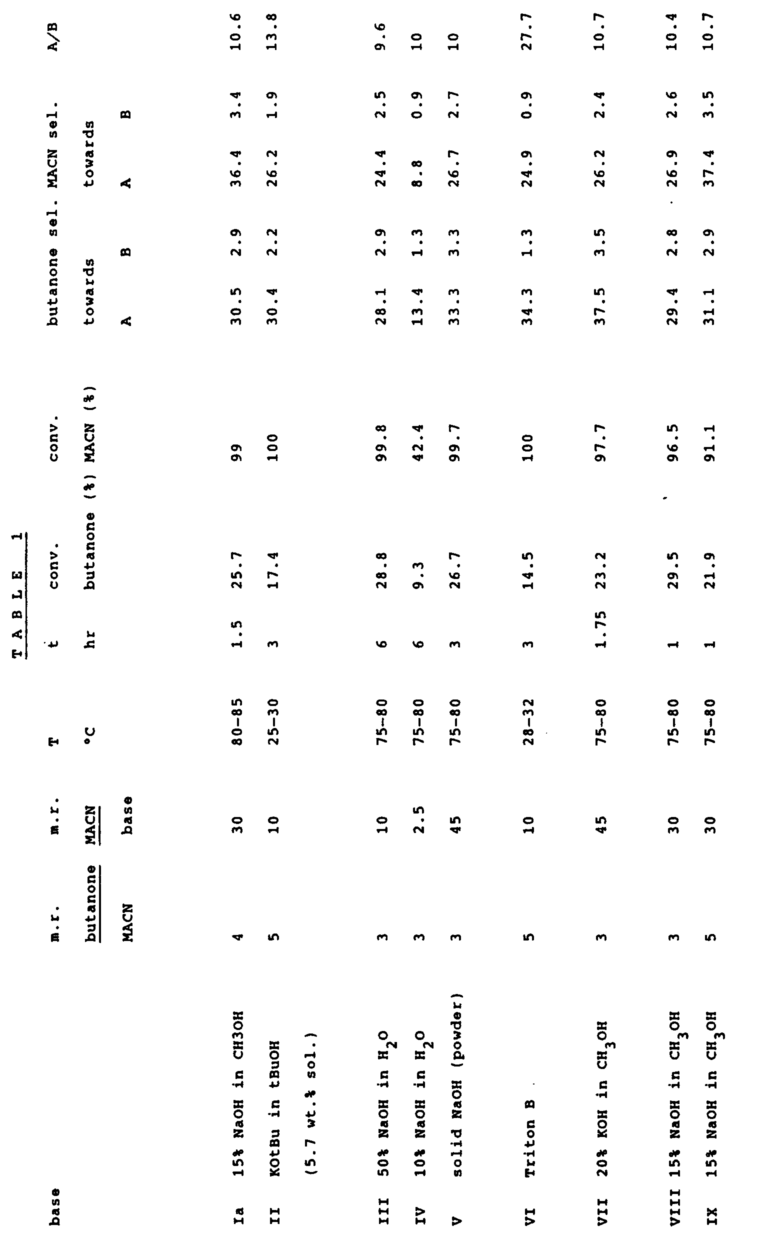

### Comparative Experiment 10

### 10.a. Preparation of A

A 2 l autoclave was charged with 538 g of butanone, 167 g of MACN, 94 g of n-propyl amine and 0.6 g of BzOH. The mixture was subsequently heated at 180 °C under autogenous pressure during 6 hours while being stirred. The pressure initially was 11 bar, decreasing to 9 bar at the end of the reaction. The reaction mixture was subsequently cooled.

According to gas chromatographic analysis about 83.3% of the MACN used had been converted and about 28.1% of the butanone used. Calculated relative to converted MACN the yield in terms of A was 36.4% and in terms of B 18.3%. Calculated relative to converted butanone these yields are about 36.1% and 18.1%, respectively (ratio of A : B = 2: 1). The results are presented in Table 2.

### 10.b. Purification

Using the rotavapor subsequently the larger part of the non-converted butanone, MACN and n-propyl amine were evaporated. After evaporation of the volatile components, 361 g of reaction mixture remained. To this mixture an equal volume (about 400 ml) of water containing about 4 wt.% HCl was added, followed by heating for 1 hour at 95 °C with stirring.

Subsequently the organic phase was separated off and the aqueous phase was extracted 4x with about 20 ml of CH₂Cl₂. The CH₂Cl₂ extracts were added to the organic phase. After drying of the organic phase over MgSO₄ and rotavapor evaporation at room temperature, 348.5 g of residue remained, which was distilled using a sieve tray column (approx. 30 practical trays) at reduced pressure. At a pressure of 12 mm Hg the main fraction came off at the top at a temperature of 100-107 °C. This way, 143.2 g of distillate was obtained. According to gas chromatographic analysis this distillate contained 108 g of A and 29.5 g of B (ratio of A : B = 3.7: 1).

### Comparative experiments 11 to 15

In a 50 ml autoclave a mixture of butanone, MACN, amine and BzOH was heated for 6 hours at a certain temperature. After cooling, the reaction mixture was analyzed gas chromatographically. The procedure was analogous to that of Comparative experiment 10a.

The results of these experiments are presented in Table 2.
These examples show that by using primary amines as a catalyst in the reaction of ketones with methacrylic acid nitrile a relatively large amount of the undesired isomer (B) is formed.

### Compartive experiment 16

A 2 l. autoclave was charged with 538 g of butanone, 132.5 g ACN, 6.36 g ethylenediamine and 0.82 g BzOH. The mixture was subsequently heated at 140°C under autogeneous pressure during 6 hours while being stirred. The reaction mixture was subsequently cooled.

According to gas chromatographic analysis about 91.8% of the ACN used had been converted and about 29.6% of the butanone used. Calculated relative to converted ACN the yield in terms of C was 69.9% and in terms of D 6.8%. Calculated relative to converted butanone these yields are about 71.7% and 7.1%, respectively (ratio C : D = 10: 1). This experiment shows that when acrylic acid nitrile is used instead of methacrylic acid nitrile, the use of primary amines as a catalyst does not result in the formation of large amounts of the undesired isomer.

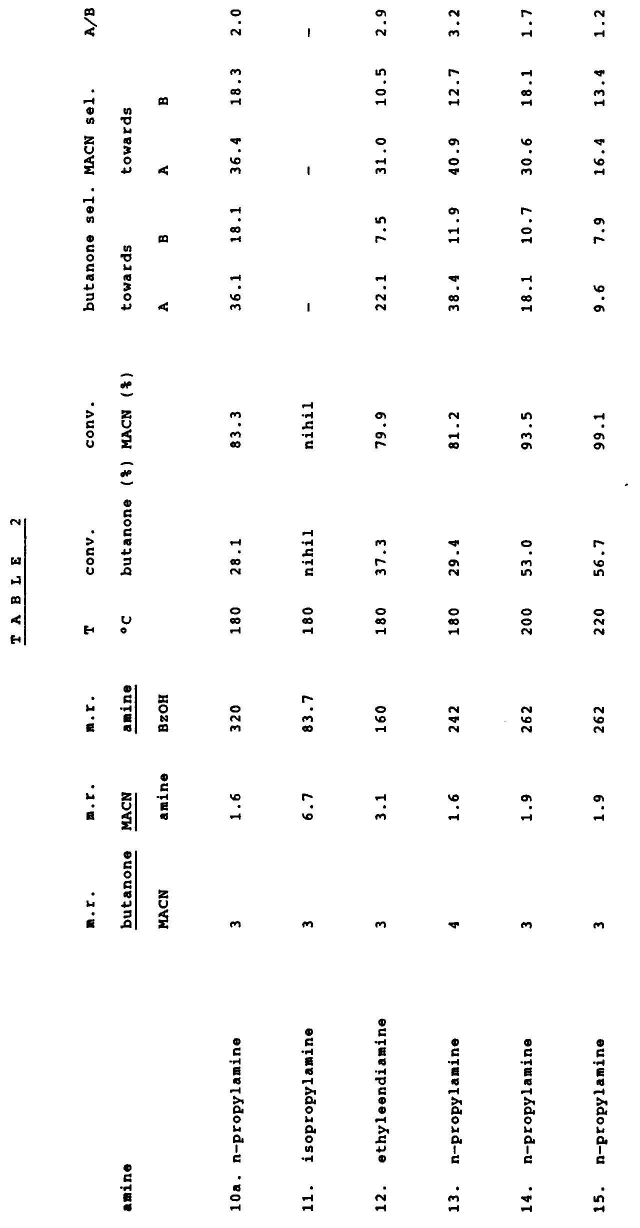

## Claims (Claims for the following Contracting State(s): AT, BE, CH, LI, DE, DK, FR, GB, IT, NL, SE)

1. Process for the preparation of 5-oxohexane nitriles according to Formula 1, 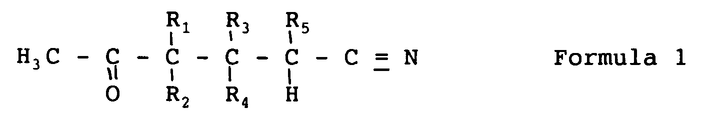 where at least R₁ or R₂ is not hydrogen, by catalytic conversion of a methyl ketone according to Formula 2 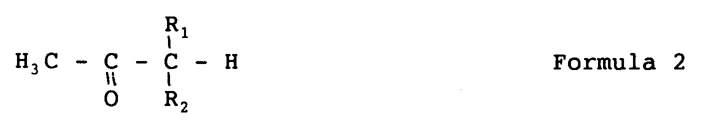 where R₁ and R₂ denote the same as in Formula 1, and an α-β unsaturated nitrile according to Formula 3 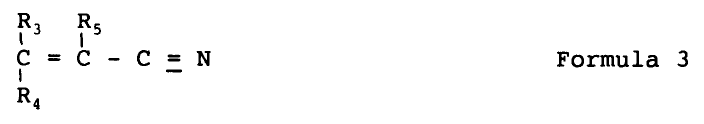 characterized in that R₁ and R₂, each independently are, H, alkyl, cycloalkyl with 1-20 carbon atoms provided that not both R₁ and R₂ are H, that R₃, R₄ and R₅, each independently are, H, alkyl, cycloalkyl with 1-20 carbon atoms provided that and that R₃, R₄ and R₅ are not all hydrogen and that the catalyst is a strong base.

2. Process according to claim 1, characterized in that the base is sodium hydroxide or potassium hydroxide.

3. Process according to either of claims 1 and 2, characterized in that the base is added as a solution in methanol.

4. Process according to claim 3, characterized in that the solution in methanol is a saturated solution.

5. Process according to either of claims 1 and 2, characterized in that the base is dissolved in water with a concentration of the base in water of more than 10 wt.%.

6. Process according to claim 1, characterized in that the base is a tetra-alkyl ammonium hydroxide.

7. Process according to any one of claims 1-6, characterized in that the molar ratio of ketone to nitrile is between 2 and 7, more preferably between 3 and 5.

8. 2,4-Dimethyl-5-oxohexane nitrile.

9. Process for the preparation of 2-methyl-3,5-dialkylpyridines wherein first the corresponding 2,4-dialkyl-5-oxohexane nitrile is prepared according to the process of any one of claims 1-7, followed by catalytic cyclization and dehydrogenation of the 2,4-dialkyl-5-oxohexane nitrile obtained.

10. Process according to claim 9, wherein 2,3,5-trimethylpyridine is prepared.

## Claims (Claims for the following Contracting State(s): ES, GR)

1. Process for the preparation of 5-oxohexane nitriles according to Formula 1, 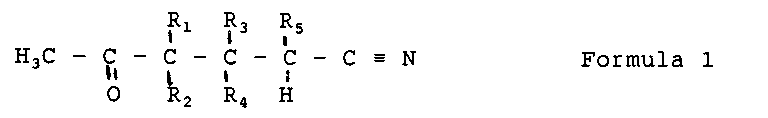 where at least R₁ or R₂ is not hydrogen, by catalytic conversion of a methyl ketone according to Formula 2 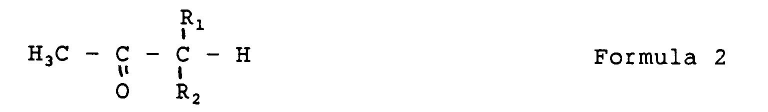 where R₁ and R₂ denote the same as in Formula 1, and an α-β unsaturated nitrile according to Formula 3 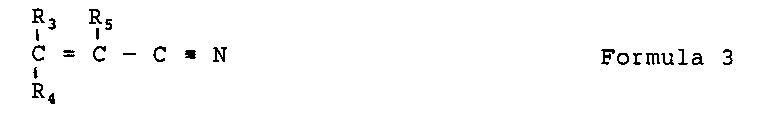 characterized in that R₁ and R₂ each independently are H, alkyl, cycloalkyl with 1-20 carbon atoms provided that not both R₁ and R₂ are H, that R₃, R₄, and R₅ each independently are H, alkyl cycloalkyl with 1-20 carbon atoms provided that R₃, R₄ and R₅ are not all hydrogen and that the catalyst is a strong base.

2. Process according to claim 1, characterized in that the base is sodium hydroxide or potassium hydroxide.

3. Process according to either of claims 1 and 2, characterized in that the base is added as a solution in methanol.

4. Process according to claim 3, characterized in that the solution in methanol is a saturated solution.

5. Process according to either of claims 1 and 2, characterized in that the base is dissolved in water with a concentration of the base in water of more than 10 wt.%.

6. Process according to claim 1, characterized in that base is a tetra-alkyl ammonium hydroxide.

7. Process according to any one of claims 1-6, characterized in that the molar ratio of ketone to nitrile is between 2 and 7, more preferably between 3 and 5.

8. Process for the preparation of 2-methyl-3,5-dialkylpyridines wherein first the corresponding 2,4-dialkyl-5-oxohexane nitrile is prepared according to the process of any one of claims 1-7, followed by catalytic cyclization and dehydrogenation of the 2,4-dialkyl-5-oxohexane nitrile obtained.

9. Process according to claim 8 wherein 2,3,5-trimethylpyridine is prepared.

## Patentansprüche (Patentansprüche für folgende(n) Vertragsstaat(en): AT, BE, CH, LI, DE, DK, FR, GB, IT, NL, SE)

1. Verfahren zur Herstellung von 5-Oxohexannitrilen gemäß Formel 1, 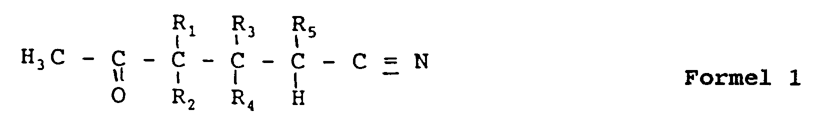 worin mindestens R₁ oder R₂ nicht Wasserstoff sind, durch katalytische Umwandlung eines Methylketons gemäß Formel 2, 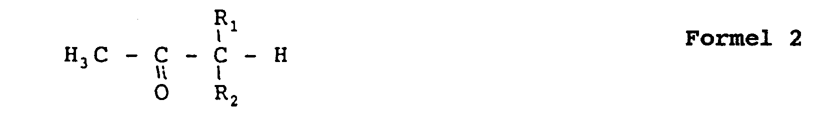 worin R₁ und R₂ die gleiche Bedeutung haben wie in Formel 1, und eines α,β-ungesättigten Nitrils gemäß Formel 3, 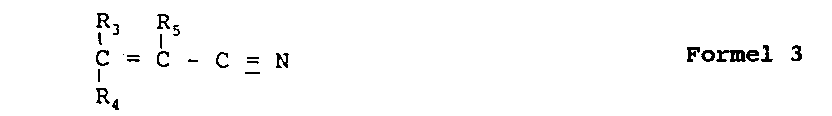 dadurch gekennzeichnet, daß R₁ und R₂ jeweils unabhängig voneinander H, Alkyl, Cycloalkyl mit 1-20 Kohlenstoffatomen sind, sofern nicht sowohl R₁ als auch R₂ H sind, daß R₃, R₄ und R₅ jeweils unabhängig voneinander H, Alkyl, Cycloalkyl mit 1-20 Kohlenstoffatomen sind, sofern R₃, R₄ und R₅ nicht alle Wasserstoff sind, und daß der Katalysator eine starke Base ist.

2. Verfahren gemäß Anspruch 1, dadurch gekennzeichnet, daß die Base Natriumhydroxid oder Kaliumhydroxid ist.

3. Verfahren gemäß einem der Ansprüche 1 und 2, dadurch gekennzeichnet, daß die Base als Lösung in Methanol zugesetzt wird.

4. Verfahren gemäß Anspruch 3, dadurch gekennzeichnet, daß die Lösung in Methanol eine gesättigte Lösung ist.

5. Verfahren gemäß einem der Ansprüche 1 und 2, dadurch gekennzeichnet, daß die Base in Wasser gelöst ist, wobei die Konzentration der Base in Wasser mehr als 10 Gew.% beträgt.

6. Verfahren gemäß Anspruch 1, dadurch gekennzeichnet, daß die Base ein Tetra-alkylammoniumhydroxid ist.

7. Verfahren gemäß einem der Ansprüche 1 bis 6, dadurch gekennzeichnet, daß das Molverhältnis von Keton zu Nitril zwischen 2 und 7, mehr bevorzugt zwischen 3 und 5 liegt.

8. 2,4-Dimethyl-5-oxohexannitril.

9. Verfahren zur Herstellung von 2-Methyl-3,5-dialkylpyridinen, worin zuerst gemäß dem Verfahren nach einem der Ansprüche 1 bis 7 das korrespondierende 2,4-Dialkyl-5-oxohexannitril hergestellt wird, gefolgt von katalytischer Cyclisierung und Dehydrogenierung des erhaltenen 2,4-Dialkyl-5-oxohexannitrils.

10. Verfahren gemäß Anspruch 9, wobei 2,3,5-Trimethylpyridin hergestellt wird.

## Patentansprüche (Patentansprüche für folgende(n) Vertragsstaat(en): ES, GR)

1. Verfahren zur Herstellung von 5-Oxohexannitrilen gemäß Formel 1, 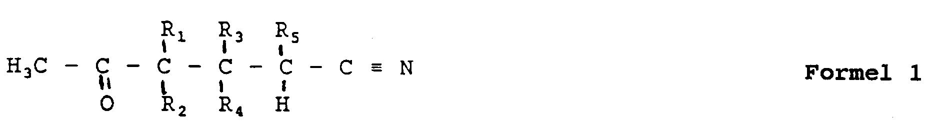 worin mindestens R₁ oder R₂ nicht Wasserstoff sind, durch katalytische Umwandlung eines Methylketons gemäß Formel 2, 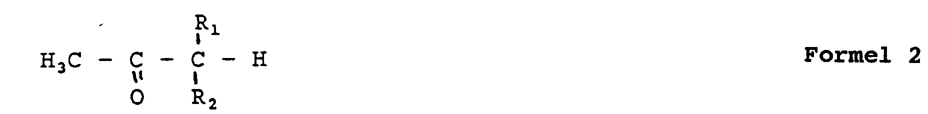 worin R₁ und R₂ die gleiche Bedeutung haben wie in Formel 1, und eines α,β-ungesättigten Nitrils gemäß Formel 3, 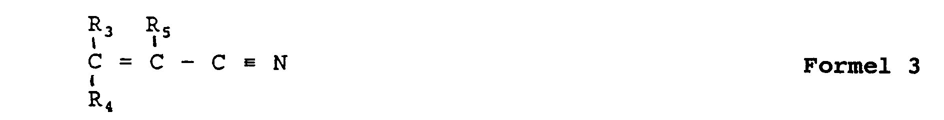 dadurch gekennzeichnet, daß R₁ und R₂ jeweils unabhängig voneinander H, Alkyl, Cycloalkyl mit 1-20 Kohlenstoffatomen sind, sofern nicht sowohl R₁ als auch R₂ H sind, daß R₃, R₄ und R₅ jeweils unabhängig voneinander H, Alkyl, Cycloalkyl mit 1-20 Kohlenstoffatomen sind, sofern R₃, R₄ und R₅ nicht alle Wasserstoff sind, und daß der Katalysator eine starke Base ist.

2. Verfahren gemäß Anspruch 1, dadurch gekennzeichnet, daß die Base Natriumhydroxid oder Kaliumhydroxid ist.

3. Verfahren gemäß einem der Ansprüche 1 und 2, dadurch gekennzeichnet, daß die Base als Lösung in Methanol zugesetzt wird.

4. Verfahren gemäß Anspruch 3, dadurch gekennzeichnet, daß die Lösung in Methanol eine gesättigte Lösung ist.

5. Verfahren gemäß einem der Ansprüche 1 und 2, dadurch gekennzeichnet, daß die Base in Wasser gelöst ist, wobei die Konzentration der Base in Wasser mehr als 10 Gew.% beträgt.

6. Verfahren gemäß Anspruch 1, dadurch gekennzeichnet, daß die Base ein Tetra-alkylammoniumhydroxid ist.

7. Verfahren gemäß einem der Ansprüche 1 bis 6, dadurch gekennzeichnet, daß das Molverhältnis von Keton zu Nitril zwischen 2 und 7, mehr bevorzugt zwischen 3 und 5 liegt.

8. Verfahren zur Herstellung von 2-Methyl-3,5-dialkylpyridinen, worin zuerst nach dem Verfahren gemäß einem der Ansprüche 1 bis 7 das korrespondierende 2,4-Dialkyl-5-oxohexannitril hergestellt wird, gefolgt von katalytischer Cyclisierung und Dehydrogenierung des erhaltenen 2,4-Dialkyl-5-oxohexannitrils.

9. Verfahren gemäß Anspruch 8, wobei 2,3,5-Trimethylpyridin hergestellt wird.

## Revendications (Revendications pour l'(les) Etat(s) contractant(s) suivant(s): AT, BE, CH, LI, DE, DK, FR, GB, IT, NL, SE)

1. Procédé pour la préparation de 5-oxohexanenitriles selon la formule 1, 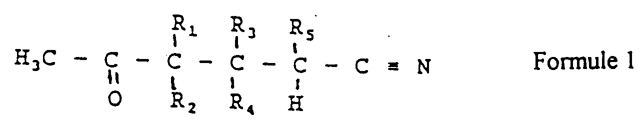 où au moins R₁ ou R₂ n'est pas l'hydrogène, par transformation catalytique d'une méthylcétone selon la formule 2 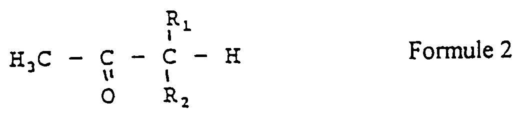 où R₁ et R₂ ont la même signification que dans la formule 1, et d'un nitrile insaturé en α-β selon la formule 3 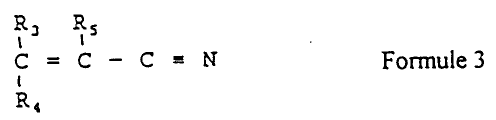 caractérisé en ce que R₁ et R₂, chacun de façon indépendante, sont H, un alkyle, un cycloalkyle avec de 1 à 20 atomes de carbone, à la condition que R₁ et R₂ ne soient pas tous les deux H, en ce que R₃, R₄ et R₅, chacun de façon indépendante, sont H, un alkyle, un cycloalkyle avec de 1 à 20 atomes de C, à la condition que R₃, R₄ et R₅ ne soient pas tous l'hydrogène, et en ce que le catalyseur est une base forte.

2. Procédé selon la revendication 1, caractérisé en ce que la base est l'hydroxyde de sodium ou l'hydroxyde de potassium.

3. Procédé selon l'une quelconque des revendications 1 et 2, caractérisé en ce que la base est ajoutée sous forme d'une solution dans le méthanol.

4. Procédé selon la revendication 3, caractérisé en ce que la solution dans le méthanol est une solution saturée.

5. Procédé selon l'une quelconque des revendications 1 et 2, caractérisé en ce que la base est dissoute dans l'eau avec une concentration de la base dans l'eau supérieure à 10 % en poids.

6. Procédé selon la revendication 1, caractérisé en ce que la base est un hydroxyde de tétra-alkylammonium.

7. Procédé selon l'une quelconque des revendications 1 à 6, caractérisé en ce que le rapport molaire entre la cétone et le nitrile est entre 2 et 7, plus préférentiellement entre 3 et 5.

8. 2,4-diméthyl-5-oxohexanenitrile.

9. Procédé pour la préparation de 2-méthyl-3,5-dialkylpyridines où l'on prépare tout d'abord le 2,4-dialkyl-5-oxohexanenitrile correspondant selon le procédé conforme à l'une quelconque des revendications 1 à 7, et en ce que ensuite on effectue une cyclisation catalytique et une déshydrogénation du 2,4-dialkyl-5-oxohexanenitrile obtenu.

10. Procédé selon la revendication 9, où l'on prépare la 2,3,5-triméthylpyridine.

## Revendications (Revendications pour l'(les) Etat(s) contractant(s) suivant(s): ES, GR)

1. Procédé pour la préparation de 5-oxohexanenitriles selon la formule 1, 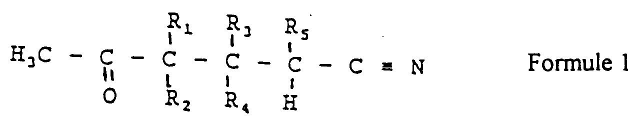 où au moins R₁ ou R₂ n'est pas l'hydrogène, par transformation catalytique d'une méthylcétone selon la formule 2 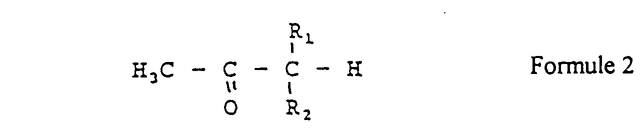 où R₁ et R₂ ont la même signification que dans la formule 1, et d'un nitrile insaturé en α-β selon la formule 3 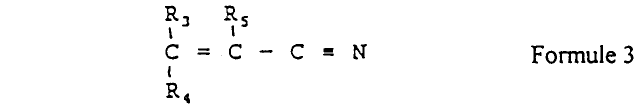 caractérisé en ce que R₁ et R₂, chacun de façon indépendante, sont H, un alkyle, un cycloalkyle avec de 1 à 20 atomes de carbone, à la condition que R₁ et R₂ ne soient pas tous les deux H, en ce que R₃, R₄ et R₅, chacun de façon indépendante, sont H, un alkyle, un cycloalkyle avec de 1 à 20 atomes de C, à la condition que R₃, R₄ et R₅ ne soient pas tous l'hydrogène, et en ce que le catalyseur est une base forte.

2. Procédé selon la revendication 1, caractérisé en ce que la base est l'hydroxyde de sodium ou l'hydroxyde de potassium.

3. Procédé selon l'une quelconque des revendications 1 et 2, caractérisé en ce que la base est ajoutée sous forme d'une solution dans le méthanol.

4. Procédé selon la revendication 3, caractérisé en ce que la solution dans le méthanol est une solution saturée.

5. Procédé selon l'une quelconque des revendications 1 et 2, caractérisé en ce que la base est dissoute dans l'eau avec une concentration de la base dans l'eau supérieure à 10% en poids.

6. Procédé selon la revendication 1, caractérisé en ce que la base est l'hydroxyde de tétra-alkylammonium.

7. Procédé selon l'une quelconque des revendications 1 à 6, caractérisé en ce que le rapport molaire entre la cétone et le nitrile est entre 2 et 7, plus préférentiellement entre 3 et 5.

8. Procédé pour la préparation de 2-méthyl-3,5-dialkylpyridines où l'on prépare tout d'abord le 2,4-dialkyl-5-oxohexanenitrile correspondant selon le procédé conforme à l'une quelconque des revendications 1 à 7, et en ce que ensuite on effectue une cyclisation catalytique et une déshydrogénation du 2,4-dialkyl-5-oxohexanenitrile obtenu.

9. Procédé selon la revendication 8, où l'on prépare la 2,3,5-trimétliylpyridine.
